# EUROPÄISCHE PATENTSCHRIFT

(11) **EP 0 733 031 B1**
(45) Veröffentlichungstag und Bekanntmachung des Hinweises auf die Patenterteilung: **08.04.1998**
(21) Anmeldenummer: 95902128.8
(22) Anmeldetag: 06.12.1994
(51) Int. Cl.: C07C 17/25, C07C 21/06, C07C 17/38

(54) **VERFAHREN UND VORRICHTUNG ZUR REINIGUNG VON VINYLCHLORID**
PROCESS AND DEVICE FOR CLEANING VINYL CHLORIDE
PROCEDE ET DISPOSITIF D'EPURATION DU CHLORURE DE VINYLE

(30) Priorität: 09.12.1993 DE 4342042
(43) Veröffentlichungstag der Anmeldung: 25.09.1996
(73) Patentinhaber: HOECHST AKTIENGESELLSCHAFT, 65926 Frankfurt am Main (DE)
(72) Erfinder: SCHWARZMAIER, Peter, D-84556 Kastl (DE); KAMMERHOFER, Peter, D-84508 Burgkirchen (DE); STÖGER, Manfred, D-84508 Burgkirchen (DE); KALLIWODA, Helmut, D-84508 Burgkirchen (DE); MIELKE, Ingolf, D-84508 Burgkirchen (DE)
(86) Internationale Anmeldenummer: EP9404050
(87) Internationale Veröffentlichungsnummer: WO9515935

(56) Entgegenhaltungen:
- DE-A- 3 024 156

## Beschreibung

Aus der US-Patentschrift 3 843 736 ist ein Verfahren bekannt zur Gewinnung von Vinylchlorid aus dem bei der unvollständigen thermischen Spaltung von 1,2-Dichlorethan bei erhöhtem Druck von vorzugsweise 8 bis 40 ata und Temperaturen zwischen etwa 450 und 650 °C in Abwesenheit von Katalysatoren anfallenden Reaktionsgemisch, wobei die die Spaltzone verlassende Gasmischung, welche aus Vinylchlorid, Chlorwasserstoff, nicht umgesetztem 1,2-Dichlorethan sowie Nebenprodukten besteht, teilweise kondensiert in eine erste Destillationsstufe zur Abdestillation des Chlorwasserstoffs und anschließend in eine zweite Destillationsstufe zur Abdestillation des Vinylchlorids geleitet wird, wonach das Sumpfprodukt der zweiten Destillationsstufe zwecks Rückführung des nicht umgesetzten 1,2-Dichlorethans in bekannter Weise aufgearbeitet wird und wobei das am Kopf der zweiten Destillationsstufe abgezogene und verflüssigte Vinylchlorid teilweise etwa in die Mitte der ersten Destillationsstufe zurückgepumpt wird, das dadurch gekennzeichnet ist, daß man das als Produktion abgezogene, verflüssigte Vinylchlorid, das noch geringe Mengen Chlorwasserstoff enthält, dem Kopf einer Entgasungszone zuführt, aus der mittels eines Umlaufverdampfers ein Teil des Vinylchlorids zusammen mit dem gesamten eingeschleppten Chlorwasserstoff abgedampft, kondensiert und etwa in die Mitte der ersten, zur Abdestillation des Chlorwasserstoffs vorgesehenen Destillationsstufe zurückgepumpt wird, während dem Boden der Entgasungszone reinstes Vinylchlorid entnommen wird.

In dieser Patentschrift wird auch erwähnt, daß die geringen Mengen des Chlorwasserstoffs, die in der Entgasungszone entfernt werden, früher mit Hilfe von Türmen, die mit Natriumhydroxid gefüllt waren, abgetrennt wurden.

Das bekannte Verfahren ist jedoch dann nachteilig, wenn das der Entgasungszone zugeführte Vinylchlorid noch geringe Wassermengen enthält. Diese sind in der Praxis unvermeidlich; vor allem beim Anfahren der Anlage und bei Betriebsunterbrechungen werden zwangsläufig Spuren von Wasser eingebracht. In der Praxis wurde deshalb dann die Entgasungszone so betrieben, daß diese Wassermengen über den Sumpf der Entgasungskolonne - zusammen mit Chlorwasserstoff - abgetrennt wurden, wofür die Natriumhydroxid-gefüllten Türme doch wieder erforderlich wurden. Würde diese Abtrennung des Wassers über den Sumpf der Entgasungskolonne nicht durchgeführt, so würde das Wasser mit dem zurückgeführten Vinylchlorid im Kreislauf geführt und in den betroffenen Anlageteilen zu Korrosionen führen. Solche Korrosionen sind jedoch nicht nur aus wirtschaftlichen, sondern vor allem aus Sicherheitsgründen nicht hinnehmbar.

Es wurde nun gefunden, daß man auf die Natriumhydroxidgefüllten Türme dann verzichten kann, wenn man die Entgasungskolonne so betreibt, daß der Großteil des Wassers über Kopf abgeführt und, zweckmäßig durch Trocknung, entfernt wird. Das in den Prozeß zurückgeführte trockene, chlorwasserstoffhaltige Vinylchlorid kann dann keine Korrosionen mehr verursachen.

Die Erfindung betrifft somit ein Verfahren zur Gewinnung von Vinylchlorid, das dadurch gekennzeichnet ist, daß aus dem bei der unvollständigen thermischen Spaltung von 1,2-Dichlorethan bei erhöhtem Druck und Temperaturen von etwa 450 bis etwa 650 °C in Abwesenheit von Katalysatoren anfallenden Reaktionsgemisch in einer ersten Destillationsstufe der Chlorwasserstoff und in einer zweiten Destillationsstufe das Vinylchlorid abdestilliert wird und wobei das am Kopf der zweiten Destillationsstufe abgezogene und verflüssigte Vinylchlorid teilweise als Rücklauf in die zweite Destillationsstufe zurückgeführt wird und das übrige abgezogene verflüssigte Vinylchlorid dem Kopf einer Entgasungszone zugeführt wird, aus der ein Teil des Vinylchlorids zusammen mit dem gesamten eingeschleppten Chlorwasserstoff und Wasser abgedampft und nach weitgehender Entfernung des Wassers in die erste Destillationsstufe zurückgeführt wird, während dem Boden der Entgasungszone reines Vinylchlorid entnommen wird.

Die Erfindung betrifft weiterhin eine Vorrichtung zur Herstellung von Vinylchlorid mit einer Spaltzone (1), einer ersten Destillationsstufe (2) für die Abtrennung von Chlorwasserstoff, einer zweiten Destillationsstufe (3) für die Abdestillation von Vinylchlorid von höhersiedenden Bestandteilen und einer dritten Destillationsstufe (4) für die Abtrennung von Chlorwasserstoff und Wasser vom Vinylchlorid, gekennzeichnet durch eine Vorrichtung (5) (Trocknungszone), über die der in der dritten Destillationsstufe (4) abgetrennte Chlorwasserstoff und Wasser mit Vinylchlorid in die erste Destillationsstufe (2) zurückgeführt und in der das Wasser weitgehend entfernt wird.

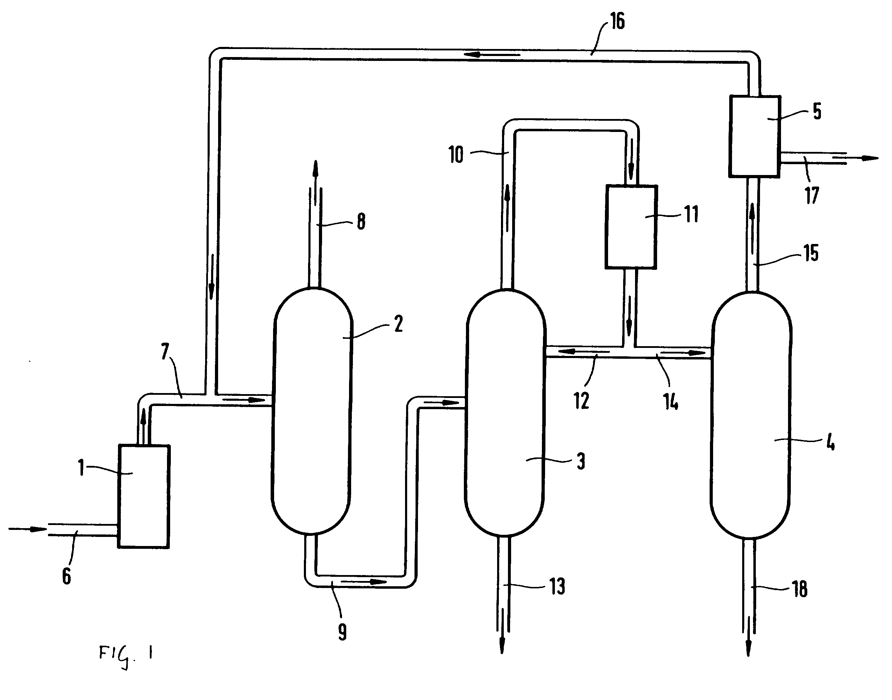

Die erfindungsgemäße Vorrichtung ist in der Figur schematisch dargestellt. In ihr bedeuten neben den bereits genannten Apparaturen (1) bis (5):
(6) die Zuleitung für 1,2-Dichlorethan zur Spaltzone (1),
(7) eine Leitung für die Spaltprodukte zur ersten Destillationsstufe (2),
(8) eine Leitung für den abgetrennten Chlorwasserstoff,
(9) eine Leitung für die höhersiedenden Produkte zur zweiten Destillationsstufe (3),
(10) eine Leitung zu einem Kondensator (11) für die Verflüssigung des Vinylchlorids,
(12) eine Leitung für einen Teilstrom des verflüssigten Vinylchlorids zurück zur zweiten Destillationsstufe (3),
(13) eine Leitung zur Abtrennung hochsiedender Nebenprodukte,
(14) eine Leitung für einen zweiten Teilstrom des verflüssigten Vinylchlorids zur dritten Destillationsstufe (4),
(15) eine Leitung von der dritten Destillationsstufe (4) zur Trocknungszone (5),
(16) eine Leitung für die getrocknete Mischung aus Chlorwasserstoff und restlichem Vinylchlorid zurück zur ersten Destillationsstufe (2),
(17) eine Leitung für das abgetrennte Wasser und
(18) eine Leitung für das reine Vinylchlorid.

Bei der erfindungsgemäßen Abtrennung des Wassers tritt in die Trocknungszone ein hochkorrosives Gemisch aus Vinylchlorid, Chlorwasserstoff und Wasser ein. Unter solchen Umständen kann bekanntermaßen eine Polymerisation des Vinylchlorids erfolgen (Katalyse durch Metallspuren). Überraschenderweise konnte jedoch auch bei längerem Betrieb in Metallapparaturen kein Polyvinylchloridbelag festgestellt werden.

Die erfindungsgemäße weitgehende Entfernung des Wassers kann in der für saure Fluide bekannten Weise erfolgen. Dies kann durch Kondensation des Wassers, vorzugsweise aber durch Trocknung, beispielsweise mit Mitteln erfolgen, die eine chemische Umwandlung erfahren, beispielsweise flüssige Trocknungsmittel wie konzentrierte Schwefelsäure oder feste Trocknungsmittel wie Phosphorpentoxid oder Calciumchlorid. Bevorzugt sind Trocknungsmittel, die das Wasser adsorptiv festhalten, wie Molekularsiebe und insbesondere Kieselgele.

Die Trocknungszone besteht vorzugsweise aus mindestens zwei parallel geschalteten Trocknern, wobei durch den einen das wasserhaltige zurückzuführende Gemisch geleitet wird, während der andere (oder die anderen) regeneriert beziehungsweise neu beschickt wird (werden). Es ist natürlich auch möglich, mehrere Trocknungsapparate in Serie zu betreiben. Die bei der Regeneration freiwerdenden Wertstoffe, Vinylchlorid und Chlorwasserstoff, können in das Verfahren zurückgeführt werden.

In einer bevorzugten Ausgestaltung der Erfindung werden die Trocknungsapparate so ausgestaltet, daß sie zum Regenerieren aufgeheizt und mit einem Inertgas wie Kohlendioxid, einem Edelgas oder vorzugsweise Stickstoff gespült werden können. Geeignet sind beispielsweise Röhren-Wärmetauscher oder Behälter mit innenliegenden Rohrschlangen.

Erfindungsgemäß kann der über Kopf der Entgasungskolonne abgeführte Gasstrom getrocknet oder das Kopfprodukt kondensiert und das Kondensat durch den beziehungsweise die Trockner geführt werden.

Zweckmäßig werden die Trockner möglichst nahe an der Entgasungszone installiert, da am Kopf dieser Kolonne die größte Wasserkonzentration herrscht - vor allem dann, wenn die Trocknung der Gasphase erfolgen soll. In diesem Falle kann der Trockner am Beginn der Brüdenleitung installiert sein.

Wenn auch erfindungsgemäß auf Natriumhydroxid-Türme und dergleichen verzichtet werden kann, so umfaßt die Erfindung selbstverständlich auch Ausführungsformen, bei denen - beispielsweise aus Sicherheitsgründen - derartige Vorrichtungen beibehalten werden. In diesem Falle haben diese Vorrichtungen dann erheblich höhere Standzeiten und einen entsprechend verringerten Anfall an Salz und Abwasser.

Wenn auf die Nachbehandlung mit Natriumhydroxid oder gleichwirkenden Mitteln verzichtet wird, werden nicht nur diese Materialien und die Apparatur eingespart, sondern es entfällt auch der Aufwand mit deren Bedienung und Wartung. Außerdem kann die gesamte Apparatur dann im geschlossenen System betrieben werden. Damit entfallen auch Emissionen beziehungsweise die zu ihrer Vermeidung dienenden Vorkehrungen beim Öffnen, Reinigen und Beschicken dieser Apparaturen.

Die Erfindung wird in den folgenden Beispielen erläutert.

### Beispiel 1

Ein Vinylchloridstrom von 39 kg/h mit einem mittleren Wassergehalt von 29,5 ppm wird durch ein Rohr (Länge 200 mm, Durchmesser 80 mm), gefüllt mit einem Drahtgewebe einer Maschendichte von 125 µm, beschickt mit 740 g Kieselgel (handelsübliches "Blaugel", engporig, 1 bis 3 mm Korngröße), gepumpt (Druck beim Eingang des Rohres 16 bar). Nach 21 h Laufdauer hat das Kieselgel 15,45 g Wasser adsorbiert, entsprechend einer Beladung von 2,1 Gew.-%. Der Wassergehalt im abströmenden Vinylchlorid beträgt 7,1 ppm.

Zur Desorption wird Niederdruckdampf durch die Apparatur geleitet. Nach 180 min beträgt die Restbeladung des Silicagels < 0,3 %.

### Beispiel 2

In einer technischen Anlage wird ein Vinylchloridstrom von 39 kg/h 19 h durch einen zylindrischen Behälter geleitet. Dimensionierung und Beschickung entsprechen im übrigen dem im Beispiel 1 beschriebenen Behälter. Nach einer Laufzeit von 19 h beträgt die Beladung des Kieselgels 3,07 Gew.-%. Der Wassergehalt des getrockneten Vinylchlorids beträgt 6,5 ppm.

Zur Regenerierung wird der Behälter auf circa 120 °C (Innentemperatur) aufgeheizt, wobei ein Stickstoffstrom von 100 l/h durch die Apparatur geleitet wird. Nach 3 h beträgt die Beladung des Kieselgels 0,25 %.

### Beispiel 3

In einer technischen Anlage wird ein Vinylchloridstrom von 34 kg/h mit einem mittleren Wassergehalt von 30 ppm durch ein Rohr (Länge 200 mm, Durchmesser 80 mm), gefüllt mit Molekularsieb (WESSALITH US 330, Firma Degussa, Hanau), mit einem Druck von etwa 16 bar gepumpt. Nach einer Laufzeit von 88 h hat das Molekularsieb 738 g Wasser adsorbiert, was einer Beladung von 31,9 Gew.-%, bezogen auf das Adsorptionsmittel, entspricht. Nach 23 h Desorption bei 250 °C beträgt die Restbeladung etwa 13 Gew.-%.

### Beispiel 4

Ein Teilstrom des Kopfstroms aus der dritten Destillationsstufe von 0,37 kg Vinylchlorid mit circa 4 g (1000 ppm) Chlorwasserstoffgehalt und circa 6 mg (16 ppm) Wassergehalt wird gasförmig abgezogen und über einen Trocknungsapparat geführt. Der Trocknungsapparat besteht aus einem Rohr der Länge 200 mm und einem Durchmesser von 15 mm. Er ist gefüllt mit 35 g Kieselgel (handelsübliches "Blaugel", engporig, 1 bis 3 mm Korngröße). Nachdem 100 l des genannten Gasstroms durch diesen Trockner geleitet wurden, betrug die Beladung des Trocknungsmittels 12,8 Gew.-%. Das austretende Gas enthielt einen durchschnittlichen Wassergehalt von 0,5 ppm.

## Patentansprüche

1. Verfahren zur Gewinnung von Vinylchlorid, dadurch gekennzeichnet, daß aus dem bei der unvollständigen thermischen Spaltung von 1,2-Dichlorethan bei erhöhtem Druck und Temperaturen von etwa 450 bis etwa 650 °C in Abwesenheit von Katalysatoren anfallenden Reaktionsgemisch in einer ersten Destillationsstufe der Chlorwasserstoff und in einer zweiten Destillationsstufe das Vinylchlorid abdestilliert wird und wobei das am Kopf der zweiten Destillationsstufe abgezogene und verflüssigte Vinylchlorid teilweise als Rücklauf in die zweite Destillationsstufe zurückgeführt wird und das übrige abgezogene verflüssigte Vinylchlorid dem Kopf einer Entgasungszone zugeführt wird, aus der ein Teil des Vinylchlorids zusammen mit dem gesamten eingeschleppten Chlorwasserstoff und Wasser abgedampft und nach weitgehender Entfernung des Wassers in die erste Destillationsstufe zurückgeführt wird, während dem Boden der Entgasungszone reines Vinylchlorid entnommen wird.

2. Verfahren nach Anspruch 1, dadurch gekennzeichnet, daß die Entfernung des Wassers mit Hilfe eines Trocknungsmittels erfolgt.

3. Verfahren nach Anspruch 2, dadurch gekennzeichnet, daß ein Trocknungsmittel eingesetzt wird, das das Wasser adsorbiert.

4. Verfahren nach Anspruch 3, dadurch gekennzeichnet, daß als Trocknungsmittel ein Molekularsieb oder Kieselgel eingesetzt wird.

5. Vorrichtung zur Herstellung von Vinylchlorid mit einer Spaltzone (1), einer ersten Destillationsstufe (2) für die Abtrennung von Chlorwasserstoff, einer zweiten Destillationsstufe (3) für die Abdestillation von Vinylchlorid von höhersiedenden Bestandteilen und einer dritten Destillationsstufe (4) für die Abtrennung von Chlorwasserstoff und Wasser vom Vinylchlorid, gekennzeichnet durch eine Vorrichtung (5) (Trocknungszone), über die der in der dritten Destillationsstufe (4) abgetrennte Chlorwasserstoff und Wasser mit Vinylchlorid in die erste Destillationsstufe (2) zurückgeführt und in der das Wasser weitgehend entfernt wird.

6. Vorrichtung nach Anspruch 5, gekennzeichnet durch einen Trockner, der mit einem Mittel beschickt ist, das Wasser physikalisch adsorbiert.

7. Vorrichtung nach Anspruch 5 oder 6, gekennzeichnet durch parallel geschaltete Trockner.

## Claims

1. A process for isolating vinyl chloride, which comprises distilling off the hydrogen chloride from the reaction mixture formed in the incomplete thermal cracking of 1,2-dichloroethane at superatmospheric pressure and temperatures of from about 450 to about 650°C in the absence of catalysts in a first distillation stage and distilling off the vinyl chloride in a second distillation stage, with the vinyl chloride drawn off at the top of the second distillation stage and liquefied being partially recirculated as runback into the second distillation stage and the remaining liquefied vinyl chloride drawn off being fed to the top of a degassing zone from which a part of the vinyl chloride together with the entire entrained hydrogen chloride and water is evaporated and, after substantial removal of the water, recirculated into the first distillation stage, while pure vinyl chloride is taken off from the bottom of the degassing zone.

2. The process as claimed in claim 1, wherein the removal of the water is carried out with the aid of a desiccant.

3. The process as claimed in claim 2, wherein the desiccant used adsorbs the water.

4. The process as claimed in claim 3, wherein the desiccant used is a molecular sieve or silica gel.

5. An apparatus for preparing vinyl chloride having a cracking zone (1), a first distillation stage (2) for separating off hydrogen chloride, a second distillation stage (3) for distilling off vinyl chloride from higher-boiling constituents and a third distillation stage (4) for separating off hydrogen chloride and water from the vinyl chloride, which comprises an apparatus (5) (drying zone) via which the hydrogen chloride and water separated off in the third distillation stage (4) are recirculated together with vinyl chloride to the first distillation stage (2) and in which the water is substantially removed.

6. The apparatus as claimed in claim 5, which comprises a dryer charged with an agent which physically adsorbs water.

7. The apparatus as claimed in claim 5 or 6, which comprises dryers connected in parallel.

## Revendications

1. Procédé d'obtention du chlorure de vinyle, caractérisé en ce l'on distille, à partir du milieu réactionnel résultant du clivage thermique incomplet du 1,2-dichloroéthane sous pression élevée et à des températures d'environ 450 à 650°C environ, en l'absence de catalyseurs, le chlorure d'hydrogène dans un premier étage de distillation et le chlorure de vinyle dans un deuxième étage de distillation et dans lequel le chlorure de vinyle extrait en tête du deuxième étage de distillation et condensé, est partiellement réintroduit à reflux dans le deuxième étage de distillation et le chlorure de vinyle condensé restant extrait est amené en tête d'une zone de dégazage, de laquelle une partie du chlorure de vinyle, en même temps que l'ensemble du chlorure d'hydrogène et de l'eau entraînés, est évaporée et réintroduite dans le premier étage de distillation après élimination essentielle de l'eau, tandis que le chlorure de vinyle pur est soutiré de la base de la zone de dégazage.

2. Procédé selon la revendication 1, caractérisé en ce que l'élimination de l'eau est effectué au moyen d'un agent desséchant.

3. Procédé selon la revendication 2, caractérisé en ce que l'on utilise un agent desséchant qui adsorbe l'eau.

4. Procédé selon la revendication 3, caractérisé en ce que l'on utilise comme agent desséchant un tamis moléculaire ou du gel de silice.

5. Dispositif de fabrication du chlorure de vinyle avec une zone de clivage (1), un premier étage de distillation (2) pour la séparation du chlorure d'hydrogène, un deuxième étage de distillation (3) pour séparer par distillation le chlorure de vinyle des constituants à plus haut point d'ébullition et d'un troisième étage de distillation (4) pour la séparation du chlorure d'hydrogène et de l'eau du chlorure de vinyle, caractérisé par un dispositif (5) (zone de séchage) à travers lequel le chlorure d'hydrogène et l'eau, séparés dans le troisième étage de distillation (4), sont réintroduits avec le chlorure de vinyle dans le premier étage de distillation (2) et dans lequel l'eau est essentiellement éliminée.

6. Dispositif selon la revendication 5, caractérisé par un sécheur qui est chargé avec un agent qui adsorbe physiquement l'eau.

7. Dispositif selon la revendication 5 ou 6, caractérisé par des sécheurs montés en parallèle.
